(19) Europäisches Patentamt
European Patent Office
Office européen des brevets

(11) Publication number: 0 204 481 A2

# (12) EUROPEAN PATENT APPLICATION

(21) Application number: 86303979.8

(22) Date of filing: 27.05.86

(51) Int. Cl.[4]: C07D 317/60

(30) Priority: 29.05.85 JP 115588/85

(43) Date of publication of application: 10.12.86 Bulletin 86/50

(84) Designated Contracting States: AT BE CH DE FR GB IT LI NL SE

(71) Applicant: SUMITOMO PHARMACEUTICALS COMPANY, LIMITED
40, Dosho-machi 2-chome
Higashi-ku Osaka-shi Osaka-fu(JP)

(72) Inventor: Ishizumi, Kikuo
16-10 Uenonishi-2-chome
Toyonaka-shi(JP)
Inventor: Maeshima, Kaoru
7-4-510 Nakakonoikecho-1-chome
Higashiosaka-shi(JP)
Inventor: Nagata, Shoji
13-1-407, Minamihibarigaoka-2-chome
Takarazuka-shi(JP)
Inventor: Kojima, Atsuyuki
21-4 Izumicho
Takarazuka-shi(JP)

(74) Representative: Paget, Hugh Charles Edward et al
MEWBURN ELLIS & CO. 2/3 Cursitor Street
London EC4A 1BQ(GB)

## (54) Process for optically active threo-3-(3,4-methylenedioxyphenyl) serine.

(57) A process for the production of optically active threo-3-(3,4-methylenedioxyphenyl)serine of formula:

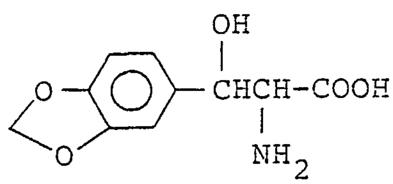

which comprises subjecting racemic threo-3-(3,4-methylenedioxyphenyl) serine alkyl ester of formula:

EP 0 204 481 A2

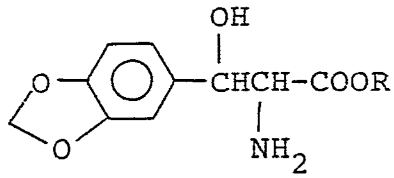

wherein R is alkyl having 1 to 4 carbon atoms to optical resolution by using a resolving agent selected from the group consisting of optically active pyroglutamic acid and optically active dibenzoyltartaric acid in a solvent medium, separating the insoluble diastereomer salt, producing D or L-threo-3-(3,4-methylenedioxyphenyl)serine alkyl ester therefrom by treatment with an aqueous alkali, and then hydrolysing it to obtain

optically active threo-3-(3,4-methylenedioxyphenyl)-serine, which is useful as intermediate for producing optically active threo-3-(3,4-dihydroxyphenyl)-serine.

# PROCESS FOR OPTICALLY ACTIVE THREO-3-(3,4-METHYLENEDIOXYPHENYL) SERINE

The present invention relates to a process for producing optically active threo-3-(3,4-methylenedioxyphenyl) serine represented by the formula

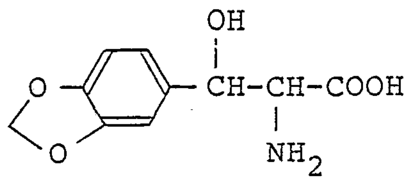

[II].

More particularly, the invention relates to a process for producing a D-or L-phenylserine derivative [II] which comprises

optical resolution of a D-and L-isomeric mixture - (including racemic form) of threo-3-(3,4-methylenedioxyphenyl) serine alkyl ester (hereinafter simply referred to as a phenylserine ester derivative) represented by the general formula

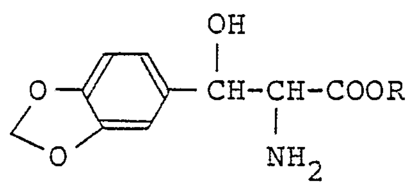

[I]

wherein R is lower alkyl, by the action of optically active pyroglutamic acid or optically active dibenzoyltartaric acid,

removing the combined carboxylic acid from the resulting carboxylate of optically active phenylserine ester derivative, and

hydrolyzing the resulting D-or L-phenylserine ester derivative [I].

As regards processes for producing the optically active phenylserine derivative [II], a process as described in U. S. Patent No. 4,480,109 is already known. As shown by reaction formula A below, this process comprises converting a racemic phenylserine derivative [II'] into N-benzyloxycarbonyl derivative thereof of formula [III} by using a benzyloxycarbonylation reagent such as benzyloxycarbonyl chloride, optical resolution - (separation of the diastereomer salts and decomposition of the salts by mineral acid treatment) by using an optically active amine such as ephedrine, quinidine, quinine or the like, and catalytic reduction of the obtained optically active N-benzyloxycarbonyl derivative in the presence of palladium on carbon or the like to yield an optically active phenylserine derivative [II].

[Reaction formula A]

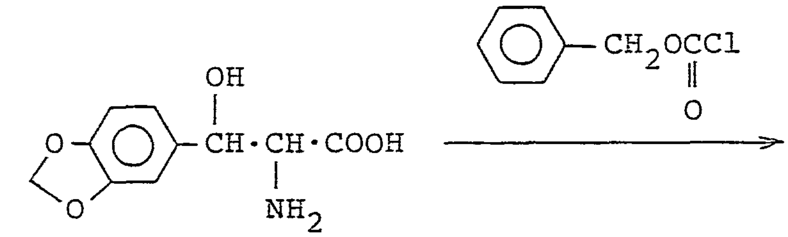

(DL)

[II']

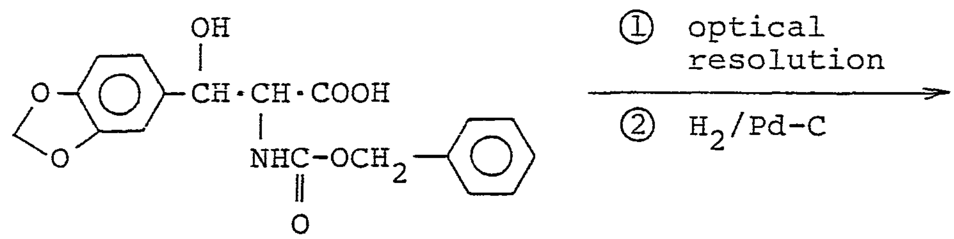


[II]

(D or L)

[III]

[Reaction formula B]

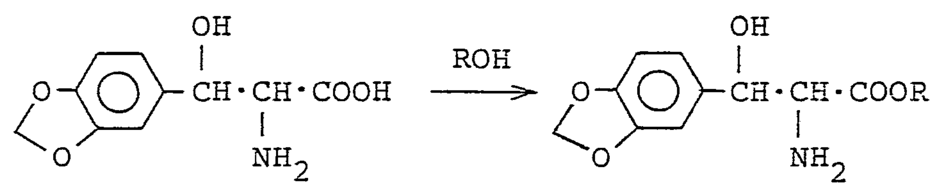


(DL)

[II']

[I]

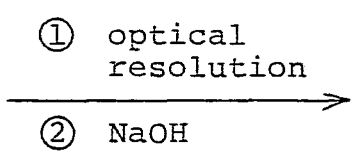


[II]

(D or L)

(In this formula, R represents lower alkyl.)

The process of the present invention, as shown by reaction formula B above, is distinguished from the known process (the process described in U.S. Patent No. 4,480,109) by forming the final product via a phenylserine ester derivative [I]. According to the present process, the phenylserine ester derivative [I] is obtained without using any of expensive reagents such as benzyloxycarbonyl chloride and palladium but using a cheap lower alcohol such as methanol, ethanol, isopropanol, or butanol and the

optically active phenylserine derivative [II] can be obtained by optical resolution of the ester [I], followed by hydrolysis of the ester portion with a cheap base such as sodium hydroxide or the like. Hence it may be said that the present process is of great industrial value.

The present inventors investigated the possibility of optical resolution of the phenylserine derivative [I] by using various optically active acids. The result showed that with optically active tartaric acid, mandelic acid, lactic acid, glutamic acid, or aspartic acid, or the like, the formed salt is noncrystalline or even when the salt is crystalline, the difference between solubilities of these two diastereomer salts in any of various solvents is not enough large to fractionate the salts by utilizing the above difference. However, it was found that pyroglutamic acid or dibenzoyltartaric acid, used in the present invention, forms two diastereomer salts between which large difference exists in solubility so that the fractionation is possible by utilizing this solubility difference. Based on this finding, the present invention has been accomplished.

The phenylserine derivative [II], which can be produced according to the process of the present invention, is useful as an intermediate for optically active threo-3-(3,4-dihydroxyphenyl)serine represented by the formula

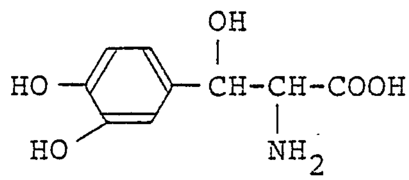

[IV].

The compound represented by formula [IV] above is known to be useful as a remedy for peripheral orthostatic hypotension (U.S. Patent No. 4,330,558), as an antidepresant (Japanese Patent Application Laid-Open No. 20747/81), and as a remedy for Parkinsonism (U.S. Patent No. 4,497,826).

In general formula [I] above, the lower alkyl group includes linear or branched $C_1$-$C_4$ alkyls, for example, methyl, ethyl, n-propyl, isopropyl, n-butyl, and t-butyl.

The present invention is described below in more detail.

According to the invention optically active threo-3-(3,4-methylenedioxyphenyl)serine represented by formula [II] can be produced as follows: A D and L isomeric mixture (including racemic form) of the phenylserine ester derivative represented by general formula [I] above is reacted with optically active pyroglutamic acid or optically active dibenzoyltartaric acid in a suitable solvent to form two diastereomer salts of the derivative. Utilizing the difference between their solubilities, the less soluble one of them is precipitated to fractionate them. Then a base is added to one of the separated diastereomer salts to split it into the salt components and obtain the desired L-or D-phenylserine ester derivative. Further, this derivative is hydrolyzed at the ester portion to yield the desired optically active threo-3-(3,4-methylenedioxyphenyl)-serine.

The slightly soluble diastereomer salt precipitated in the above process, for instance, when dibenzoyl-L-tartaric acid is reacted on the phenylserine ester derivative of general formula [I] wherein R is methyl, is the salt of L-phenylserine ester derivative with dibenzoyl-L-tartaric acid, while the salt of D-phenylserine ester derivative with dibenzoyl-L-tartaric acid remains in the mother liquor.

In the optical resolution step of the present inventive process, the diastereomer salts can be formed and fractionated at temperatures of 0 to 80°C or by heating a solution of the salts up to around the boiling point of the solvent and the cooling to a temperature of 0 to 30°C. In view of optical resolution efficiency, preferred temperatures of the salt formation and fractionation are 20 to 40°C. The salt formation is satisfactory in several minutes though several days may be taken for it. There is no particular restriction on the salt formation period.

Optically active pyroglutamic acid or optically active dibenzoyltartaric acid, an optically resolving agent, is used in an amount of 0.5 to 1 mole per mole of a D and L isomeric mixture (including racemic form) of the phenylserine ester derivative. Preferably the amount is from 0.8 to 1 mole in view of optical resolution efficiency.

Suitable solvents for use to form and fractionate the above salts include; alcohols, e.g. methanol, ethanol, and isopropanol; dioxane, acetone, water, and mixtures of these solvents. Dioxane is preferred in view of optical resolution efficiency.

The obtained salt of optically active phenylserine ester derivative [I] with optically active pyroglutamic acid or with optically active dibenzoyltartaric acid is split by treatment with a basic

aqueous solution, and the precipitated crystals are taken by filtration or by extraction with an organic solvent. Thus the optically active phenylserine ester derivative [I] can be obtained.

Suitable bases for the above basic aqueous solution are, for example, sodium hydroxide, potassium hydroxide, sodium carbonate, potassium carbonate, and sodium hydrogencarbonate. The salt can be split according to the ordinary method by using the above base in an amount equivalent to the salt or more.

Suitable organic solvents for the extraction include, for example, ethyl acetate chloroform, dichloroethane, dichloromethane, and diethyl ether.

It is also possible to prepare a mineral acid salt of optically active phenylserine ester derivative [I] by adding an aqueous mineral acid solution to the above salt to split this, and washing the resulting aqueous solution with an organic solvent, followed by concentrating the aqueous solution.

For example, hydrochloric acid, sulfuric acid, or phorphoric acid is used as the mineral acid in an amount of 1 to 2 equivalents to the salt.

Suitable organic solvents for the washing are, for example, ethyl acetate, chloroform, dichloroethane, dichloromethane, and diethyl ether.

Conventional conditions of hydrolyzing esters are applicable to the hydrolysis of the optically active phenylserine ester derivative [I] obtained by the salt splitting. However, the hydrolysis is better carried out in a solution of an alkali or alkaline earth metal hydroxide (e.g. sodium hydroxide, potassium hydroxide, barium hydroxide etc.) in a polar solvent such as methanol, ethanol, water, or a mixture of them at room temperature or under cooling. After completing of the reaction, the objective product can be isolated and purified according to techniques used for the isolation and purification of common amino acids.

Since the objective compound of the present inventive process is an amino acid, it is possible, if desired, to transform the amino group into an inorganic salt of hydrochloric acid, sulfuric acid, or the like or into an addition salt of an organic acid such as acetic acid, methanesulfonic acid, or the like, or transform the carboxylic group into an alkaline salt, alkaline earth salt or ammonium salt.

The phenylserine ester derivative represented by general formula [I], which is used as a starting material in the process of the present invention, can be prepared, as shown in reaction formula B above, by subjecting a D and L isomeric mixture - (including racemic form) of the phenylserine derivative (II') (see U.S. Patent No. 4,480,109) to esterification.

Conventional conditions of esterifying amino acids can be applied to this esterification. For instance, the phenylserine derivative (II') can be esterified in an excess of the corresponding alcohol in the presence of an acid catalyst.

Optically active threo-3-(3,4-methylenedioxyphenyl)serine (II), produced according to the process of the present invention, is readily phthaloylated with so-called Nefkens' reagent (N-carboethoxyphthalamide) to yield optically active threo-N-phthaloyl-3-(3,4-methylenedioxyphenyl)serine [V]. From this compound [V], useful optically active threo-3-(3,4-dihydroxyphenyl)serine [IV] can be derived by the method described in European Patent Application No. 128684. Optically active threo-3-(3,4-dihydroxyphenyl)serine [IV] can also be obtained by the reaction of optically active threo-3-(3,4-methylenedioxypphenyl)serine [II] according to the method described in U.S. Patent No. 4,480,109.

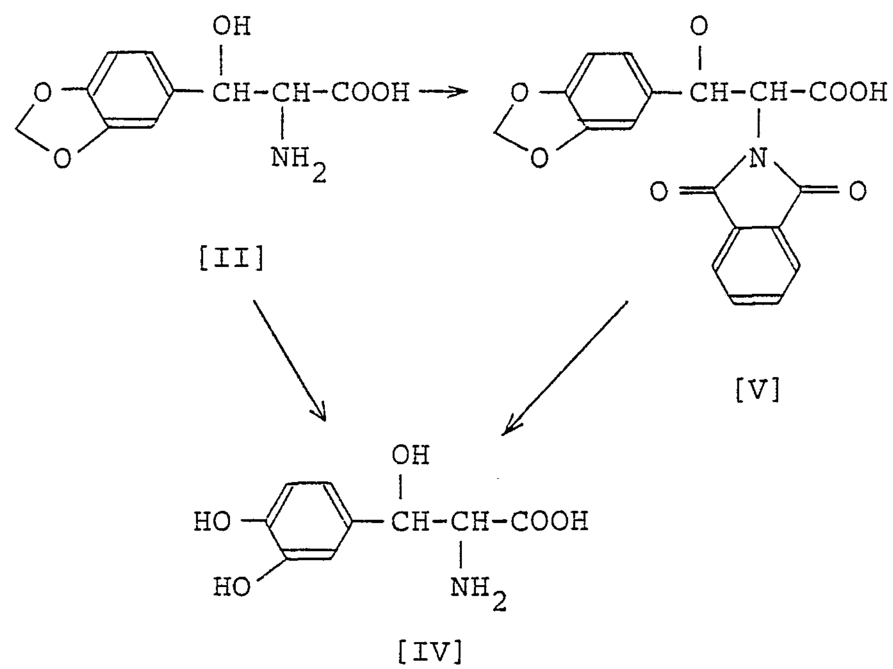

[II] [V] [IV]

The following examples and reference examples illustrate the present invention but do not restrict it.

Reference Example 1 Preparation of DL-threo-3-(3,4-methylenedioxyphenyl)serine methyl ester

1 Methanol (740 ml) was added to DL-threo-3-(3,4-methylenedioxyphenyl)serine acetate (100 g). While cooling the mixture at 0°C or below, thionyl chloride (74 ml) was added dropwise with stirring. Thereafter, the mixture was heated to 50 -60°C, stirred for 5 hours, and then concentrated in vacuo. To the residue was added again methanol (300 ml), and the mixture was concentrated in vacuo. After repeating this procedure three times, isopropanol (300 ml) was added to the residue, and the mixture was left standing overnight. The precipitated crystals were filtered to yield DL-threo-3-(3,4-methylenedioxyphenyl)serine methyl ester hydrochloride, m.p. 155°C (decomp.).

2 This hydrochloride (20 g) was added to a 4.8% aqueous sodium hydrogencarbonate solution (210 g). The mixture was stirred fo 5 hours under ice cooling. The precipitated crystals were filtered to yield DL-threo-3-(3,4-methylenedioxyphenyl)-serine methyl ester, m.p. 128 -129°C.

IR absorption ($cm^{-1}$) in liquid paraffin:

3400, 1730, 1600, 1300, 1280, 1250, 1210, 1160, 1130, 1080, 1020, 1000, 920, 860

Reference Example 2 Preparation of L-threo-N-phthaloyl-3-(3,4-methylenedioxyphenyl)serine

1 L-Threo-3-(3,4-methylenedioxyphenyl)serine - (2 g) and anhydrous sodium carbonate (1.13 g) were dissolved in water (20 ml). To this solution was added N-carbethoxyphthalimide (2.73 g) at a temperature of up to 5°C. This mixture was stirred at room temperature for 3 hours and then made slightly acidic by adding conc. hydrochloric acid. The precipitated crystals were filtered to give L-threo-N-phthaloyl-3-(3,4-methylenedioxyphenyl)-serine (3.06 g), m.p. 90 -96°C.

$[\alpha]_D$ = -91.4° (C=1, methanol)

Example 1 Preparation of L-threo-3-(3,4-methylenedioxyphenyl)serine methyl ester

1 DL-Threo-3-(3,4-methylenedioxyphenyl)serine methyl ester (0.50 g) and dibenzoyl-L-tartaric acid monohydrate (0.79 g) were dissolved in methanol - (5 ml). This solution was left standing for 72 hours at 0 -5°C. The precipitated crystals were filtered to yield L-threo-3-(3,4-methylenedioxyphenyl)serine methyl ester • dibenzoyl-L-tartarate, m.p. 136°C - (decomp.).

$[\alpha]_D^{25}$ = -86.8° (C=1, methanol)

To a portion (0.20 g) of the above salt were added saturated aqueous sodium chloride (5 ml), and sodium hydrogencarbonate (0.14 g). The mixture was extracted twice with ethyl acetate (10 ml each) and dried over anhydrous sodium sulfate.

The solvent was removed in vacuo to give L-threo-3-(3,4-methylenedioxyphenyl)serine methyl ester, m.p. 132 - 133°C.

$[\alpha]_D^{25}$ = +21.2° (C=1, methanol)

Example 2 Preparation of D-threo-3-(3,4-methylenedioxyphenyl)serine methyl ester

1 DL-Threo-3-(3,4-methylenedioxyphenyl)serine methyl ester (0.5 g) and L-pyroglutamic acid (0.27 g) were dissolved in isopropanol (20 ml). This solution was left standing for 12 hours at room temperature. The precipitated crystals were filtered to yield D-threo-3-(3,4-methylenedioxyphenyl)-serine methyl ester • L-pyroglutamate, m.p. 116 - 118°C.

$[\alpha]_D^{25}$ = +1.7° (C=1, methanol)

2 To a portion (0.1 g) of the above salt were added saturated aqueous sodium chloride (3 ml) and sodium hydrogencarbonate (0.07 g). The mixture was extracted twice with ethyl acetate (5 ml each) and dried over anhydrous sodium sulfate. The solvent was removed in vacuo to give D-threo-3-(3,4-methylenedioxyphenyl)serine methyl ester, m.p. 131 -133°C.

$[\alpha]_D^{25}$ = -21.3° (C=1, methanol)

Example 3 Preparation of L-threo-3-(3,4-methylenedioxyphenyl)serine

L-Threo-3-(3,4-methylenedioxyphenyl)serine methyl ester (3 g) obtained in Example 1 was added to methanol (6 ml), and a 5N aqueous sodium hydroxide solution (4 ml) was added dropwise to the mixture with stirring under ice cooling. Continuing this cooling, the mixture was stirred for 2 further hours and then the pH was adjusted with conc. hydrochloric acid to about 5.5. The precipitated crystals were filtered to yield L-threo-3-(3,4-methylenedioxyphenyl)serine, m.p. 191°C - (decomp.).

$[\alpha]_D$ = -29.8° (C=1, N-HCL)

Example 4 Preparation of L-threo-3-(3,4-methylenedioxyphenyl)serine methyl ester

1 DL-Threo-3-(3,4-methylenedioxyphenyl)serine methyl ester (20 g) and dibenzoyl-L-tartaric acid monohydrate (31.6 g) were dissolved in dioxane - (160 ml). This solution was left standing for 5 hours at room temperature. The precipitated crystals were filtered to yield L-threo-3-(3,4-methylenedioxyphenyl)serine methyl ester • dibenzoyltartarate - (20.8 g), m.p. 138°C (decomp.).

$[\alpha]_D^{25}$ = -75.4° (C=1, methanol)

2 A portion of the above salt was decomposed in the same manner as in Example 1 to yield L-threo-3-(3,4-methylenedioxyphenyl)serine methyl ester, m.p. 132-134°C.

$[\alpha]_D^{25}$ = +23.2° (C=1, methanol)

## Claims

1. A process for the production of optically active threo-3-(3,4-methylenedioxyphenyl)serine of the formula:

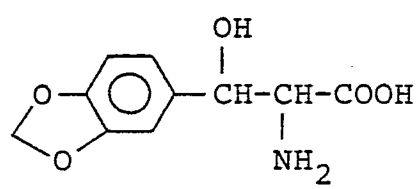

which comprises subjecting D and L isomeric mixture of threo-3-(3,4-methylenedioxyphenyl)serine alkyl ester of the formula:

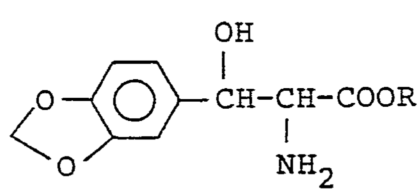

wherein R is alkyl having 1 to 4 carbon atoms to optical resolution by using a resolving agent selected from the group consisting of optically active pyroglutamic acid and optically active dibenzoyltartaric acid in a solvent medium, separating the insoluble diastereomer salt, producing D or L-threo-3-(3,4-methylenedioxyphenyl)serine alkyl ester therefrom by treatment with a basic aqueous solution, and then hydrolysing.

2. The process according to claim 1, wherein R is methyl.

3. The process according to claim 1 or claim 2, wherein a resolving agent is used in an amount of 0.8 to 1 mole per 1 mole of racemic threo-3-(3,4-methylenedioxyphenyl)serine alkyl ester.

4. The process according to any one of claims 1 to 3 wherein the optical resolution is carried out in a solvent selected from alcohols of 1 to 4 carbon atoms, acetone, dioxane tetrahydrofuran and aqueous mixtures thereof.

5. The process according to any one of claims 1 to 4 wherein the D and L isomeric mixture of threo-3-(3,4-methylenedioxyphenyl)serine alkyl ester is racemic form.

6. A process for the production of optically active threo-3-(3,4-methylenedioxyphenyl)serine alkyl ester of the formula:

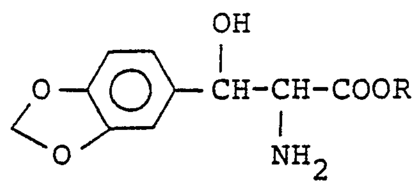

wherein R is alkyl having 1 to 4 carbon atoms which comprises subjecting racemic threo-3-(3,4-methylenedioxyphenyl)serine alkyl ester to optical resolution by using a resolving agent selected from the group consisting of optically active pyroglutamic acid and optically active dibenzoyltartaric acid in a solvent medium, separating the insoluble diastereomer salt, and treating the resulting diastereomer salt with an aqueous alkali.

7. The process according to claim 6, wherein R is methyl.

8. The process according to claim 6 or claim 7, wherein a resolving agent is used in an amount of 0.8 to 1 mole per 1 mole of racemic threo-3-(3,4-methylenedioxyphenyl)serine alkyl ester.

9. The process according to any one of claims 6 to 8 wherein the optical resolution is carried out in a solvent selected from alcohols of 1 to 4 carbon atoms, acetone, dioxane tetrahydrofuran and aqueous mixtures thereof.

10. The process according to any one of claims 6 to wherein the D and L isomeric mixture of threo-3-(3,4-methylenedioxyphenyl)serine alkyl ester is racemic form.

11. D or L-threo-3-(3,4-methylenedioxyphenyl)-serine alkyl ester.

12. A compound according to claim 12, wherein the alkyl is methyl.